# EUROPÄISCHE PATENTSCHRIFT

(11) **EP 0 158 747 B1**
(45) Veröffentlichungstag und Bekanntmachung des Hinweises auf die Patenterteilung: **12.01.1994**
(21) Anmeldenummer: 84201933.3
(22) Anmeldetag: 24.12.1984
(51) Int. Cl.: C07D 307/89, C07D 307/60, B01J 19/00

(54) **Verfahren zum periodischen Beheizen des Produktabscheiders einer Anlage zur Herstellung von Phthalsäureanhydrid oder Maleinsäureanhydrid**
Process for periodically heating the product separator of a plant for the production of phthalic anhydride and maleic anhydride
Procédé pour le chauffage périodique du séparateur de produit d'une installation pour préparer de l'anhydride phtalique ou de l'anhydride maléique

(30) Priorität: 30.03.1984 DE 3411732
(43) Veröffentlichungstag der Anmeldung: 23.10.1985
(73) Patentinhaber: METALLGESELLSCHAFT AG, 60015 Frankfurt (DE)
(72) Erfinder: Franz, Volker, D-6000 Frankfurt am Main (DE); Geissen, Rolf, D-6000 Frankfurt am Main (DE)
(74) Vertreter: Rieger, Harald, Dr.

(56) Entgegenhaltungen:
- EP-A- 0 035 173
- EP-A- 0 052 745
- DE-A- 1 926 244
- DE-A- 3 130 986
- DE-B- 2 255 400
- Lexikon der Energietechnik und Kraftmaschinen, Band 7 (1965), Seite 539

## Beschreibung

Die Erfindung betrifft ein Verfahren zum periodischen Beheizen und Kühlen des Abscheiders einer Anlage zur Herstellung von Phthalsäureanhydrid (PSA) oder Maleinsäureanhydrid (MSA), wobei man dem Abscheider in der Kühlphase dampfförmiges Produkt zuführt, ihn in einen Kühlkreislauf einschaltet und das Produkt an Kühlflächen des Abscheiders niederschlägt, in der Beheizungsphase den Abscheider in einen Wärmeträger-Flüssigkeit enthaltenden Heizkreislauf einschaltet, die Kühlflächen indirekt aufheizt, das Produkt abschmilzt und es in flüssiger Form aus dem Abscheider leitet, und für den Wärmebedarf Wasserdampf in zeitlich weitgehend konstanter Menge verwendet, der in der Anlage im Überschuß anfällt.

Bei der üblichen Ausführung der Abscheidung des in der Gasphase erzeugten PSA- oder MSA-Rohproduktes wird das aus der Produktionsanlage kommende Gas abgekühlt und überwiegend durch Desublimation an der Außenseite eines indirekt gekühlten Wärmeaustauschers abgeschieden. Das Austragen des abgeschiedenen Rohproduktes erfolgt durch Abschmelzen, wobei zumeist dieselbe Wärmeträger-Flüssigkeit wie in der Kühlphase, üblicherweise ein Wärmeträgeröl, bei einer Temperatur, die oberhalb des Produktschmelzpunktes liegt, durch den Wärmeaustauscher geleitet wird. Die Wärmeträger-Flüssigkeit wird außerhalb des Produktabscheiders im Wärmeträger-Erhitzer aufgeheizt, der mit Wasserdampf beheizt wird. Ein Verfahren dieser Art ist in der EP-A- 35 173 beschrieben. Zum Ausgleich des stark schwankenden Wärmebedarfs wird hier für die Wärmeträger-Flüssigkeit ein dem Heizkreislauf überlagerter Speicherkreislauf vorgesehen, dem man periodisch Wärmeträger-Flüssigkeit entnimmt. Allgemeine Erläuterungen zu Wasserdampfspeichern finden sich im "Lexikon der Energietechnik und Kraftmaschinen", L-Z, Band 7, Seite 539.

Der Erfindung liegt die Aufgabe zugrunde, den Ausgleich des schwankenden Wärmebedarfs auf möglichst einfache und wirtschaftliche Weise durchzuführen.

Beim eingangs genannten Verfahren besteht die Lösung der Aufgabe darin, daß man
a) die Wärmeträger-Flüssigkeit in einem Wärmeaustauscher (Wärmeträger-Erhitzer) mit Wasserdampf aus der Anlage indirekt erhitzt,
b) zu Beginn des Abschmelzvorgangs während der Zeitspanne des größten Dampfbedarfs dem Wärmeträger-Erhitzer ein Gemisch aus zwei Wasserdampfströmen zuführt, wobei der eine Strom direkt aus der Anlage kommt und der andere, größere Strom aus einem Wasserdampfspeicher herangeführt wird, und
c) den Wasserdampfspeicher während der Zeitspanne, in der der Wärmeträger-Erhitzer außer Betrieb ist oder mit geringer Last betrieben wird, mit Wasserdampf aus der Anlage belädt.

Die Menge des in der Gesamtanlage erzeugten Wasserdampfes ist üblicherweise größer als der gesamte Dampfverbrauch innerhalb der Anlage, so daß ständig eine bestimmte Menge an überschüssigem Wasserdampf zur Verfügung steht und zu anderen Zwecken genutzt werden kann. Wichtig ist hierbei, daß die Menge des nach außen abgegebenen Wasserdampfs möglichst wenig schwankt, da sich die Verbraucher an solche Schwankungen nur sehr schwer anpassen können und nur diejenige Dampfmenge wirtschaftlich nutzbar ist, die ständig zur Verfügung steht.

Durch den erfindungsgemäßen Einsatz des Dampfspeichers wird es möglich, die Menge des aus der Anlage zur Erzeugung von PSA oder MSA herausgeführten Wasserdampfs mit Schwankungen von höchstens ± 5 % und üblicherweise höchstens ± 2 % konstant zu halten, so daß auch eine weitgehend konstante Menge an Wasserdampf für andere Verbraucher zur Verfügung steht.

Der Wasserdampfspeicher ist ein bekanntes Aggregat, in welchem heißes Wasser unter Druck gehalten wird, wobei durch geregeltes Entspannen eine gewünschte Dampfmenge entnommen werden kann. Beim erfindungsgemäßen Verfahren schwankt der Druck im Dampfspeicher zumeist etwa innerhalb des Bereichs von 3 bis 40 bar. Da die Schmelztemperatur des PSA- oder MSA-Produktes weit unterhalb der Siedetemperatur des voll geladenen Wasserdampfspeichers liegt, kann der größte Teil der zum Aufheizen und Abschmelzen benötigten Wärmemenge dem Wärmeträger-Erhitzer auf einem relativ niedrigen Temperaturniveau zugeführt werden. Hieraus ergibt sich ein kleines spezifisches Speichervolumen und geringe Abmessungen des Behälters des Wasserdampfspeichers.

Ein weiterer Vorteil des Verfahrens besteht darin, daß die Wärmeträger-Flüssigkeit nur auf relativ niedrige Temperaturen erhitzt wird, so daß auch kostengünstige Öle auf Mineralölbasis verwendet werden können.

Eine Möglichkeit der Ausgestaltung des Verfahrens wird mit Hilfe der Zeichnung erläutert.
- Fig. 1: zeigt das Schema der Verfahrensführung und
- Fig. 2: einen Vergleich des Wasserdampfverbrauchs.

Dem Reaktor 1 zum Erzeugen von PSA wird durch die Leitung 2 das Gemisch der Ausgangsstoffe, Luft aus der Leitung 3 und Orthoxylol oder Naphthalin aus der Leitung 4 zugeführt. Soll MAS erzeugt werden, sind die Ausgangsstoffe Luft und Benzol oder niedrigsiedende Kohlenwasserstoffe (C₄-Schnitt). Da diese Syntheseverfahren bekannt sind, braucht hier nicht näher darauf eingegangen zu werden. Zum indirekten Kühlen der stark exothermen Umsetzung im Reaktor 1 dient eine Salzschmelze, die in der Leitung 5 in den Reaktor eintritt und ihn in der Leitung 6 verläßt. Die Salzschmelze wird im Wärmeaustauscher 7 mittels Speisewasser aus der Leitung 8 gekühlt; der dabei entstehende Wasserdampf wird in der Leitung 9 abgeführt.

Das erzeugte Reaktionsgas, ein Gemisch aus dampfförmigem Produkt (PSA oder MSA) und Luft, verläßt den Reaktor 1 in der Leitung 10 und wird in einem Kühler 11 ebenfalls mit Speisewasser einer ersten indirekten Kühlung unterzogen, der dabei entstehende Wasserdampf kann ebenfalls in die Leitung 9 eingespeist werden. Vorgekühltes Reaktionsgas wird in der Leitung 12 zum weiteren Kühlen und Verfestigen des Produkts dem Abscheider 13 zugeführt.

Zu einem Reaktor 1 gehören üblicherweise mindestens 3 Abscheider 13, denen wechselweise produkthaltiges Reaktionsgas aus der Leitung 12 zugeführt wird. In der Zeichnung ist zur Vereinfachung jedoch nur ein Abscheider dargestellt. Zum Kühlen des Abscheiders 13 und zum Niederschlagen des Produkts an den Kühlflächen dient Kühlflüssigkeit, die in der Leitung 14 herangeführt wird und den Abscheider 13 in der Leitung 15 verläßt. Der Kühlflüssigkeitskreislauf steht unter der Wirkung der Kreislaufpumpe 16 und führt über einen Kühler 17. Eine Stichleitung 18 verbindet den Kühlflüssigkeitskreislauf mit einem Ausdehnungsgefäß 19. Die Temperatur der Kühlflüssigkeit in der Leitung 14 liegt üblicherweise im Bereich von 45 bis 60^{o}C und in der Leitung 15 im Bereich von 50 bis 70^{o}C.

Als Kühlflüssigkeit dient üblicherweise ein Öl, das auch als Wärmeträger-Flüssigkeit zum Aufheizen des Abscheiders 13 und zum Abschmelzen des niedergeschlagenen Produkts verwendet wird. In der Heizphase ist die Zufuhr von Reaktionsgas aus der Leitung 12 zum Abscheider 13 unterbrochen und auf andere, nicht dargestellte Abscheider gelenkt. Gleichzeitig ist die Zufuhr von Kühlflüssigkeit abgestellt.

Nunmehr tritt Wärmeträger-Flüssigkeit mit einer Temperatur im Bereich von 140 bis 210^{o}C in der Leitung 21 in den Abscheider 13 ein, bringt das dort niedergeschlagene Produkt zum Abschmelzen, wobei das Produkt durch die Leitung 22 in einen Tank 23 fließt. Zum Flüssigkeitskreislauf, der den Abscheider 13 aufheizt, gehört die Rückleitung 24 mit der Kreislaufpumpe 25 sowie die Stichleitung 26 mit dem Ausdehnungsgefäß 27. Die Temperatur der Flüssigkeit in der Leitung 24 liegt üblicherweise im Bereich von 100 bis 200^{o}C. Im Wärmeträger-Erhitzer 28 wird die Wärmeträger-Flüssigkeit, üblicherweise ein Öl, mit Wasserdampf aus der Leitung 30 indirekt erhitzt. Abgekühlter Wasserdampf und Kondensat verlassen den Erhitzer 28 durch Leitung 31.

Zu Beginn der Aufheizphase im Abscheider 13, wenn im Erhitzer 28 ein großer Wärmebedarf besteht, kommt der größere Teil des benötigten Wasserdampfs aus dem Dampfspeicher 32 und strömt durch die Leitung 33, in der sich ein Rückschlagventil 34 befindet, und durch Leitung 30 zum Erhitzer 28.

In der Leitung 35 wird eine zeitlich konstante Wasserdampfmenge herangeführt, wobei die Konstanz des Durchflusses in einem Durchflußmesser 36 überwacht und mit Hilfe einer Regelung 37 und eines Regelventils 38 eingestellt wird. Der Druck in der Leitung 35 liegt üblicherweise im Bereich von 15-40 bar.Während der ersten Aufheizphase, wenn der Dampfverbrauch im Erhitzer 28 groß und der Druck dort entsprechend niedrig ist, strömt der Wasserdampf aus der Leitung 35 in die Leitung 40 und vermischt sich mit dem aus der Leitung 33 kommenden Wasserdampf des Dampfspeichers 32. Gegen Ende der Aufheizphase, wenn der Wasserdampfverbrauch abgenommen hat und der Druck im Erhitzer 28 angestiegen ist, wird durch eine Druckregelung 41 das Regelventil 42 in der zum Dampfspeicher 32 führenden Leitung 43 geöffnet und der Speicher 32 geladen.

Sobald die kondensierte Dampfmenge im Erhitzer 28 die aus der Leitung 40 herangeführte Wasserdampfmenge unterschreitet, steigt der Druck auf der Dampfseite des Erhitzers an, und aus dem Dampfspeicher 32 wird kein weiterer Dampf mehr entnommen. Das druckgesteuerte Regelventil 42 sowie das Rückschlagventil 34 verhindern zunächst, daß der weiter zuströmende Wasserdampf in den Speicher 32 gelangt. Dadurch steigt der Dampfdruck im Erhitzer 28 wie gewünscht in kurzer Zeit auf seinen Maximalwert. Erst wenn dieser Maximalwert erreicht ist und die zuströmende Wasserdampfmenge den Bedarf an dieser Stelle überschreitet, wird das Regelventil 42 so weit geöffnet, daß der Dampfdruck im Erhitzer 28 weiterhin gehalten wird. Die überschüssige Wasserdampfmenge gelangt nunmehr durch die Leitung 43 in den Speicher 32 und heizt diesen unter ständiger Druckerhöhung auf.

Wenn der Abschmelzvorgang im Abscheider 13 beendet ist, wird der Abscheider durch Umschalten auf den Kühlkreislauf (Leitungen 14 und 15, Pumpe 16 und Kühler 17) wieder rückgekühlt. Der Dampfverbrauch im Wärmeträger-Erhitzer 28 geht gegen Null, die zuströmende Wasserdampfmenge wird fast vollständig vom Speicher 32 aufgenommen, bis überall stromab von der Leitung 35 der gleiche Druck erreicht ist.

Für die Produktabscheidung genügen ein Dampfspeicher 32 und ein Erhitzer 28, denen mehrere Abscheider 13 wechselweise zugeschaltet werden.

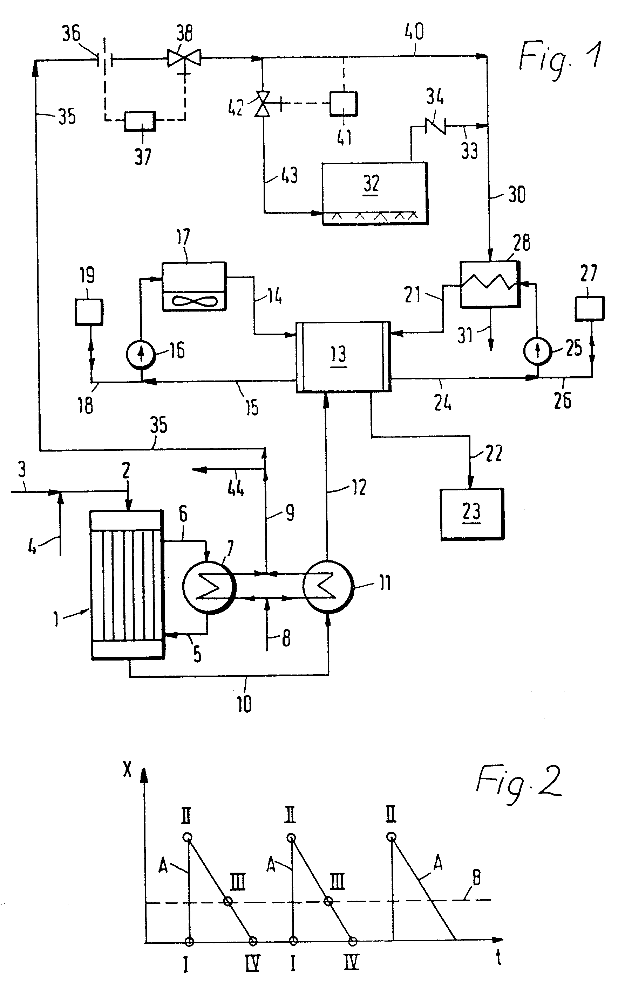

Das Diagramm der Figur 2 zeigt in Gegenüberstellung den zeitlichen Verlauf des Verbrauchs an Wasserdampf X im Erhitzer 28 in Form der Sägezahnlinie A und den in der Leitung 35 herangeführten Wasserdampf mit seinem zeitlich konstanten Verlauf (gestrichelte Linie B). Durch Zwischenschalten des Wasserdampfspeichers 32 gelingt es, trotz starker Verbrauchsspitzen (Linie A) mit in der Menge konstanter Anlieferung von Wasserdampf auszukommen. Da nur ein Teil des in der Leitung 9 geförderten Wasserdampfs in der Leitung 35 weitergeführt wird, kann in der Leitung 44 eine konstante Wasserdampfmenge an andere Verbraucher abgegeben werden.

Wenn man, wie üblich, mit mindestens 3 Abscheidern arbeitet, gehört jede der drei Sägezahnspitzen der Figur 2 zu einem anderen Abscheider. Zum Zeitpunkt I wird einer der Abscheider auf Abschmelzen geschaltet und unmittelbar darauf, zum Zeitpunkt II, wird die maximale Wasserdampfmenge im Erhitzer 28 verbraucht. Zum Zeitpunkt III braucht der Dampfspeicher 32 keinen Wasserdampf mehr abzugeben, der Druck im Speicher und im Erhitzer 28 hat zu diesem Zeitpunkt sein Minimum erreicht. Zum Zeitpunkt IV wird die Beheizung des jeweils auf Abschmelzen geschalteten Abscheiders beendet.

### Beispiel:

In einer der Figur 1 entsprechenden Verfahrensführung wird folgendermaßen gearbeitet:
Pro Stunde verlassen den Reaktor 1 durch die Leitung 10 81 000 kg eines Gemisches aus dampfförmigem Roh-PSA und Luft mit einer Temperatur von 380^{o}C. Nach Vorkühlung im Kühler 11 strömt das Gemisch mit einer Temperatur von 170^{o}C in einen von vier Abscheidern 13. Durch die Wärmeabfuhr im KÜhler 11 und Wärmeaustauscher 7 können pro Stunde in der Leitung 9 24 000 kg Wasserdampf, bei dem es sich um Sattdampf von 20 bar handelt, abgeführt werden. Von dieser Menge Sattdampf werden 10% (2 400 kg/h) durch die Leitung 35 in den Erhitzer 28 und/oder den Wasserdampfspeicher 32 geleitet, der Rest kann in der Leitung 44 anderen Verbrauchern zugeführt werden.

Zum Kühlen oder Beheizen der Abscheider dient ein übliches Wärmeträgeröl auf Mineralölbasis. Die pro Stunde durch die Leitung 14 oder 21 fließende Menge dieses Öls beträgt 110 000 kg. Der sich zeitlich ändernde Wärmebedarf zum Abschmelzen des Produkts in einem Abscheider führt zu schwankenden Werten, die in der nachfolgenden Tabelle angegeben sind. I bedeutet der Zeitpunkt unmittelbar vor dem Beginn des Beheizens des Abscheiders, benachbarte Zeitpunkte I, vgl. Figur 2 der Zeichnung, liegen etwa 2 Stunden auseinander. Die Zeitpunkte II, III und IV haben die bereits zusammen mit Figur 2 erläuterte Bedeutung. P ist der Druck, Q ist die Durchfußmenge und T die Temperatur in der jeweiligen Leitung. Der Druck in den Leitungen 33 und 30 ist gleich dem Druck in der Leitung 40.

**Tabelle**

| Zeitpunkt | | | | |
|---|---|---|---|---|
| Leitung | I | II | III | IV |
| 40 | P = 19 bar | P = 18 bar | P = 6 bar | P = 19 bar |
| | Q = 100 kg/h | Q = 2400 kg/h | Q = 24oo kg/h | Q = 100 kg/h |
| 33 | Q = - | Q = 5600 kg/h | Q = - | Q = - |
| 43 | P = 18 bar | P = 18 bar | P = 6 bar | P = 8 bar |
| | Q = 2300 kg/h | Q = - | Q = - | Q = 2300 kg/h |
| 30 | Q = 100 kg/h | Q = 8000 kg/h | Q = 2400 kg/h | Q = 100 kg/h |
| 14 | T = 55^{o}C | - | - | - |
| 15 | T = 70^{o}C | - | - | - |
| 21 | - | T = 140^{o}C | T = 150^{o}C | T = 190°C |
| 24 | - | T = 100^{o}C | T = 135^{o}C | T = 185^{o}C |

## Patentansprüche

1. Verfahren zum periodischen Beheizen und Kühlen des Abscheiders einer Anlage zur Herstellung von Phthalsäureanhydrid (PSA) oder Maleinsäureanhydrid (MSA), wobei man dem Abscheider in der Kühlphase dampfförmiges Produkt zuführt, ihn in einen Kühlkreislauf einschaltet und das Produkt an Kühlflächen des Abscheiders niederschlägt, in der Beheizungsphase den Abscheider in einen Wärmeträger-Flüssigkeit enthaltenden Heizkreislauf einschaltet, die Kühlflächen indirekt aufheizt, das Produkt abschmilzt und es in flüssiger Form aus dem Abscheider leitet, und für den Wärmebedarf Wasserdampf in zeitlich weitgehend konstanter Menge verwendet, der in der Anlage im Überschuß anfällt, dadurch gekennzeichnet, daß man
a) die Wärmeträger-Flüssigkeit in einem Wärmeaustauscher (Wärmeträger-Erhitzer) mit Wasserdampf aus der Anlage indirekt erhitzt,
b) zu Beginn des Abschmelzvorgangs während der Zeitspanne des größten Dampfbedarfs dem Wärmeträger-Erhitzer ein Gemisch aus zwei Wasserdampfströmen zuführt, wobei der eine Strom direkt aus der Anlage kommt und der andere, größere Strom aus einem Wasserdampfspeicher herangeführt wird, und
c) den Wasserdampfspeicher während der Zeitspanne, in der der Wärmeträger-Erhitzer außer Betrieb ist oder mit geringer Last betrieben wird, mit Wasserdampf aus der Anlage belädt.

2. Verfahren nach Anspruch 1, dadurch gekennzeichnet, daß man die Menge des aus der Anlage herangeführten Wasserdampfs mit Schwankungen von höchstens ± 5 % konstant hält.

3. Verfahren nach Anspruch 1 oder 2, dadurch gekennzeichnet, daß der Wasserdampfspeicher erst nach Erreichen des Maximaldruckes im Wärmeträger-Erhitzer beladen wird.

## Claims

1. A process for the periodic heating and cooling of the separator of an installation for producing phthalic acid anhydride (PA) or maleic anhydride (MA), in which vaporous product is fed to the separator in the cooling phase, said separator is switched into a cooling circuit and the product is deposited on cooling surfaces of the separator, in the heating phase the separator is switched into a heating circuit containing liquid heat-transfer medium, the cooling surfaces are indirectly heated, the product melts down and is passed out of the separator in liquid form, and for the heat consumption steam which is produced as a surplus in the installation is used at a rate which is largely constant over time, characterised in that
a) the liquid heat-transfer medium is heated indirectly in a heat-exchanger (heater for the heat-transfer medium) with steam from the installation,
b) at the beginning of the melting-down operation during the period of the maximum steam requirement, a mixture of two streams of steam is fed to the heater for the heat-transfer medium, one stream coming directly from the installation and the other, larger stream being brought in from a steam accumulator, and
c) the steam accumulator is charged with steam from the installation during the period in which the heater for the heat-transfer medium is out of operation or is operated at a low load.

2. A process according to Claim 1, characterised in that the quantity of the steam brought in from the installation is kept constant with fluctuations of at most ± 5%.

3. A process according to Claim 1 or 2, characterised in that the steam accumulator is not charged until the maximum pressure has been reached in the heater for the heat-transfer medium.

## Revendications

1. Procédé de chauffage et de refroidissement périodique du séparateur d'une installation de préparation d'anhydride phtalique (PSA) ou d'anhydride maléique (MSA), qui consiste à envoyer au séparateur dans la phase de refroidissement du produit sous forme de vapeur, à le brancher dans un circuit de refroidissement et à précipiter le produit sur les surfaces de refroidissement du séparateur, à brancher dans la phase de chauffage le séparateur dans un circuit de chauffage contenant du liquide caloporteur, à chauffer indirectement les surfaces de refroidissement, à fondre le produit et à l'évacuer sous forme liquide du séparateur, et à utiliser pour la chaleur dont on a besoin la vapeur d'eau en une quantité sensiblement constante dans le temps, cette quantité se formant en excès dans l'installation, caractérisé en ce qu'il consiste
a) à chauffer indirectement, par de la vapeur d'eau provenant de l'installation, le liquide caloporteur dans un échangeur de chaleur (dispositif de chauffage à échangeur de chaleur),
b) à envoyer au début du processus de fusion, pendant l'intervalle de temps où les besoins en vapeur sont les plus grands, au dispositif de chauffage du caloporteur, un mélange de deux courants de vapeur d'eau, l'un des courants provenant directement de l'installation et l'autre, plus grand, d'une réserve de vapeur d'eau, et
c) à charger de vapeur d'eau, provenant de l'installation, la réserve de vapeur d'eau pendant l'intervalle de temps pendant lequel le dispositif de chauffage du caloporteur est hors de fonctionnement ou fonctionne à faible charge.

2. Procédé suivant la revendication 1, caractérisé en ce qu'il consiste à maintenir constante la quantité de vapeur d'eau amenée de l'installation avec des fluctuations de ± 5 % au plus.

3. Procédé suivant la revendication 1 ou 2, caractérisé en ce qu'il consiste à charger la réserve de vapeur d'eau seulement après avoir obtenu la pression maximum dans le dispositif de chauffage du caloporteur.
